# EUROPÄISCHE PATENTSCHRIFT

(11) **EP 1 827 317 B1**
(45) Veröffentlichungstag und Bekanntmachung des Hinweises auf die Patenterteilung: **17.06.2009**
(21) Anmeldenummer: 05857848.5
(22) Anmeldetag: 23.08.2005
(51) Int. Cl.: A61F 2/28, A61F 2/78, A61L 27/30

(54) **SUBKUTANES, INTRAMUSKULÄRES LAGER FÜR EIN STARRES TRANSKUTANES IMPLANTAT**
SUBCUTANEOUS INTRAMUSCULAR MOUNTING FOR A RIGID TRANSCUTANEOUS IMPLANT
SUPPORT INTRAMUSCULAIRE SOUS-CUTANE POUR UN IMPLANT TRANSCUTANE RIGIDE

(30) Priorität: 11.11.2004 DE 102004055623
(43) Veröffentlichungstag der Anmeldung: 05.09.2007
(73) Patentinhaber: ESKA Implants GmbH & Co. KG, 23556 Lübeck (DE)
(72) Erfinder: GRUNDEI, Hans, 23558 Lübeck (DE)
(74) Vertreter: Fuchs
(86) Internationale Anmeldenummer: PCT/EP2005/009075
(87) Internationale Veröffentlichungsnummer: WO 2006/053593

(56) Entgegenhaltungen:
- EP-A- 1 559 383
- DE-A1- 10 040 590
- DE-B3- 10 247 397
- US-A- 4 158 895

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem Implantat und einer daran ankoppelbaren extrakorporalen Koppelungseinrichtung aufweist. Das Zwischenstück ist als starre Buchse mit einem in Richtung intrakorporal geschlossenen Ankopplungselement ausgebildet, an das die extrakorporalen Kopplungseinrichtung koppelbar ist.

Ein derartiges Lager ist bekannt aus der DE 102 47 397 B3. Es weist einen auf die Außenwandung der Buchse aufgebrachten Schlauch aus flexiblem Material und auf den flexiblen Schlauch aufgebrachte metallische Wolle auf. Hiermit wird das Ziel verfolgt, dass die Sicherheit gegen eine Verkeimung der Durchtrittsstelle des Implantates und der angrenzenden Bereiche des Oberschenkelstumpfes deutlich erhöht wird und dass ein versehentliches Entfernen der Keimschranke verhindert wird, wie dies bei einem Lager gemäß der DE 100 40 590 A1 beispielsweise bei der Reinigung der Durchtrittsstelle des Implantates durch beispielsweise den Oberschenkelstumpf durch ein unbeabsichtigtes Durchstechen des flexiblen Materials, in den meisten Fällen Silikon, mit einer Kanüle geschehen kann.

Die Erhöhung der Sicherheit gegenüber einer Verkeimung wird bei dem gattungsgemäßen Lager deutlich dadurch erhöht, dass in die metallische Wolle umgebendes Gewebe eingranuliert. Dies funktioniert deutlich bei schlanken und muskulösen Patienten. Bei einer anderen Patientengruppe, nämlich der adipösen Patienten, können hier Schwierigkeiten auftreten, da insofern das Implantat von viel Fettgewebe, jedoch wenig Muskeln umgeben wird. Fettgewebe aber granuliert praktisch nicht in die metallische Wolle ein. Bei diesen Patienten kommt es zu einer Reizung des das Implantat umgebenden Gewebes, keinesfalls aber zu einer Durchwachsung der metallischen Wolle. Der ständige Reibeeffekt stellt eine Verkeimungsgefahr bei dieser Patientengruppe dar.

Vor diesem Hintergrund ist es nun die Aufgabe der vorliegenden Erfindung, ein subkutanes intramuskuläres Lager so weiterzubilden, dass die Sicherheit gegen eine Verkeimung der Durchtrittsstelle des Implantates und der angrenzenden Bereiche des Oberschenkelstumpfes vor allem bei adipösen Patienten nochmals deutlich erhöht wird.

Gelöst wird diese Aufgabe dadurch, dass sich die Buchse von ihrer extrakorporal auszurichtenden Seite hin zu ihrer intrakorporal auszurichtenden Seite stark aufweitet und eine glatte Oberfläche aufweist. Die das Zwischenstück bzw. die Buchse umgebenden Weichteile arthrophieren an die Buchse heran, und zwar im intrakorporal zugewandten Bereich der Buchse deutlich stärker als im extrakorporal zugewandten Teil aufgrund der speziellen Ausbildung der Buchse. Dies bedeutet eine stetig zunehmende Abdichtung vom extrakorporal zugewandten Teil der Buchse hin zum intrakorporal zugewandten Teil. Dies ist auch von umgebendem Fettgewebe zu erwarten, so dass also auch adipöse Patienten optimal versorgt werden können.

Die Aufweitung der Hülse erfolgt in einem Maße im Verhältnis von 1:1,2 bis 1:2, wenn die Länge der Basiskante der Buchse am extrakorporal ausgerichteten Ende zu 1 gesetzt wird. Hierdurch wird ein ausreichender "Sektkorkeneffekt" der Buchse hinsichtlich der Abdichtung erzielt.

Gemäß einer besonders bevorzugten Ausführungsform wirkt die Oberfläche der Buchse antibakteriell. Hierzu kann sie gemäß Ausführungsformen versilbert oder titanisiert sein.

Gemäß einer besonders bevorzugten Ausführungsform weist das Lager ein Adaptionsrohr auf, das in das Innere der Buchse greift und dort lösbar in einem Presssitz sitzt, in welches die ankoppelbare Koppeleinrichtung setzbar ist, mit antibakterieller Wirkung zumindest an seiner Außenwandung. Durch die Verwendung des Adaptionsrohres wird eine besonders hohe Keimsperre erzielt.

Nimmt die antibakterielle Wirkung seiner Außenwandung im Laufe der Zeit ab, so kann es nach Lösung des Presssitzes durch ein neues, "frisches" Adaptionsrohr ersetzt werden. Hierzu muss lediglich die extrakorporale Koppelungseinrichtung vom Zwischenstück abgekoppelt werden und das Adaptionsrohr herausgezogen werden.

In bevorzugter Weiterbildung besteht das Adaptionsrohr aus massivem Silber. Die antibakterielle Wirkung von Silber ist allgemein bekannt.

Gemäß einer anderen Ausführungsform besteht das Adaptionsrohr aus einem Material, welches auf seiner Außenwandung versilbert ist. Diese Ausführungsform ist kostengünstiger als die vorstehend erwähnte. Beispielsweise kann als Basismaterial eine Kobaltchrommolybdänlegierung Anwendung finden.

Alternativ kann das Adaptionsrohr aus einem Material bestehen, welches auf seiner Außenwandung mit Hydroxylapatit, Kalziumphosphat, Titan oder Plasmatitanspray beschichtet ist. Die vorerwähnten Beschichtungsmaterialien haben die Eigenschaft, dass Haut und Bindegewebe sich an die Außenwandung anlagern und so auf der Länge des Adaptionsrohres für eine zusätzliche keimhemmende Wirkung sorgen. Gleichwohl wächst ein eventuell vorhandenes Bindegewebe nicht in die Oberfläche des Rohres hinein, sondern lagert sich lediglich an, so dass die Lösbarkeit des Adaptionsrohres zum Zwecke des Ersatzes weiterhin gewährleistet bleibt.

Alternativ kann das Adaptionsrohr gemäß einer noch weiteren Ausführungsform aus Polyurethan bestehen. Polyurethan wird in der Medizintechnik als antibakterielles Material eingesetzt.

Gemäß einer besonderen Ausführungsform ist vorgesehen, dass das Adaptionsrohr eine solche Länge aufweist, dass es mit seiner distalen Stirnkante auf einer Schulter aufsitzt, die an der extrakorporalen Koppelungseinrichtung ausgebildet ist, wenn diese am Ankoppelungselement angekoppelt ist. Hierdurch wird ein sauberer Übergang von der extrakorporalten Koppelungseinrichtung hin zum Zwischenstück erzielt und Verschmutzungen bzw. Verkeimungen des Inneren des Adaptionsrohres weitgehend verhindert.

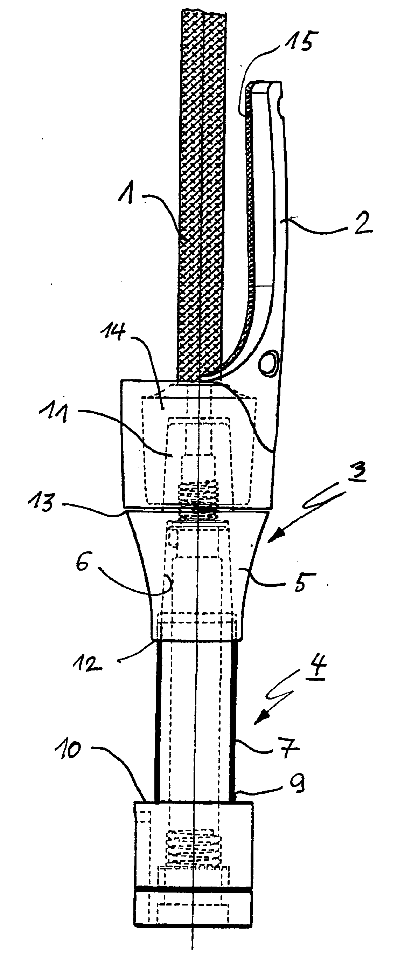

Die Erfindung wird anhand der einzigen Zeichnungsfigur beispielhaft näher erläutert. Diese zeigt im Schnitt das Zwischenstück mit angekoppeltem Adaptionsrohr sowie die extrokorporale Koppelungsvorrichtung.

Das Zwischenstück 3 ist als Buchse 5 ausgebildet und sitzt zwischen dem transkutanen Implantat (1), mit weichem es über seinen Steckkonus 11 verbindbar ist, und einer daran ankoppelbaren extrakorporalen Koppelungseinrichtung 4.

Im Inneren der Buchse 5 ist ein in Richtung intrakorporal, in der Zeichnungsfigur also nach oben, geschlossenes Ankoppelungselement 6 vorgesehen, vorliegend als konische Klemmhülse angedeutet. An das Ankoppelungselement 6 ist die extrakorporale Koppelungseinrichtung 4 koppelbar.

Wie deutlich erkennbar ist, weitet sich die Buchse 5 von Ihrem extrakorporal auszurichtenden Ende 12 hin zu ihrem intrakorporalen Ende 13 stark auf und weist eine glatte Oberfläche auf. Die Aufweitung vom extrakorporalen zum intrakorporalen Ende kann, muss aber nicht linear erfolgen. Im vorliegenden Fall ist eine leichte Krümmung dargestellt.

Hierbei ist im dargestellten Ausführungsbeispiel das Verhältnis der Länge der Buchse 5 an ihrem extrakorporal zuzuwendenden Ende 12 zu der Länge an ihrem intrakorporal zuzuwendenden Ende 13 ca. 2,3:3,6 = 1:1,6. Diese Verhältnis kann liegen in dem Intervall zwischen 1:1,2 und 1:2, um einen hinreichenden Abdichteffekt des arthrophierenden umliegenden (Fett-)Gewebes zu erzielen. Die Oberfläche der Buchse ist hierbei glatt, so dass kein irgendwie geartetes Gewebe in die Oberfläche einwachsen kann, auf der anderen Seite aber auch keine Reizung durch Reibeffekte erfährt, was einer Verkeimung Vorschub leisten würde.

Bevorzugt ist die Oberfläche der Buchse antibakteriell wirksam. Hierfür kann sie versilbert sein oder titanisiert sein.

In das Innere der Buchse 5 greift das Adaptionsrohr 7. Es sitzt in dem Inneren der Buchse 5 in einem lösbaren Presssitz, damit es bei einem notwendigen Austausch entfernt und durch ein neues Adaptionsrohr ersetzt werden kann. In dieses Adaptionsrohr 7 ist die extrakorporale Koppelungseinrichtung 4 setzbar. Vorliegend sind die Dimensionen so gewählt, dass bei angekoppelter extrakorporafer Koppelungseinrichtung 4 die distale Stirnkante 9 des Adaptionsrohres 7 auf der Schulter 10, die an der Koppelungseinrichtung 4 ausgebildet ist, aufsitzt, wenn die Koppelungseinrichtung 4 an das Zwischenstück 3 angekoppelt ist. Hierdurch wird ein Formenschluss erzielt, der eine Verschmutzung des Inneren des Adaptionsrohres 7 unterbindet.

Dargestellt ist noch eine am Implantat 1 angeformte Osteosyntheseplatte, die sich vorzugsweise von ventral an die Kortikalis des Knochenstumpfes (nicht dargestellt) anlegt. Die innenseitig vorgesehene offenmaschige dreidimensionale Raumnetzstruktur 15 verwächst mit der Kortikalis, so dass der Verbleib des Implantates 1 im Knochenstumpf langfristig stabil bleibt. Die Osteosyntheseplatte 2 hat die Aufgabe, am Übergang des Stieles des Implantates hin zu seiner Adapterhülse 14 auftretende Kräfte in die Kortikalis einzuleiten. Die Adapterhülse 14 nimmt den Steckkonus 11 des Zwischenstückes 3 auf.

### Bezugszeichenliste

- 1: transkutanes Implantat
- 2: Osteosyntheseplatte
- 3: Zwischenstück
- 4: extrakorporale Koppelungseinrichtung
- 5: Buchse
- 6: Ankoppelungselement
- 7: Adaptionsrohr

- 9: distale Stirnkante
- 10: Schulter
- 11: Steckkonus
- 12: extrakorporal ausgerichtetes Ende der Buchse
- 13: intrakorporal ausgerichtetes Ende der Buchse
- 14: Adapterhülse
- 15: 3D-Raumnetzstruktur

## Patentansprüche

1. Subkutanes, intramuskuläres Lager für ein starres transkutanes Implantat, welches intrakorporal in einem Knochenstumpf verankerbar ist, das ein Zwischenstück (3) zwischen dem Implantat und einer daran ankoppelbaren extrakorporalen Kopplungseinrichtung (4) aufweist, bei dem das Zwischenstück (3) als starre Buchse (5) mit einem in Richtung intrakorporal geschlossenen Ankoppelungselement (6) ausgebildet ist, an das die extrakorporale Koppelungseinrichtung (4) koppelbar ist,
**dadurch gekennzeichnet, dass**
sich die Buchse (5) von ihrem extrakorporal auszurichtenden Ende (12) hin zu ihrem intrakorporal auszurichtenden Ende (13) stark aufweitet und eine glatte Oberfläche aufweist.

2. Lager nach Anspruch 1, bei dem die Buchse eine Basiskante aufweist und die Länge der Basiskante der Buchse (5) an ihrem extrakorporal auszurichtenden Ende (12) sich verhält zu der Länge an ihrem intrakorporal auszurichtenden Ende (13) wie zwischen 1:1,2 bis 1:2.

3. Lager nach Anspruch 1 oder 2, bei dem die Oberfläche der Buchse (5) antibakteriell wirkt.

4. Lager nach Anspruch 3, bei dem die Oberfläche der Buchse (5) versilbert ist.

5. Lager nach Anspruch 3, bei dem die Oberfläche der Buchse (5) titanisiert ist.

6. Lager nach einem der Ansprüche 1 bis 5 mit einem Adaptionsrohr (7), das in das Innere der Buchse (5) greift und dort lösbar in einem Presssitz sitzt, in welches die ankoppelbare Koppelungseinrichtung (4) setzbar ist, mit antibakterieller Wirkung zumindest an seiner Außenwandung.

7. Lager nach Anspruch 6, bei dem das Adaptionsrohr (7) aus Silber besteht.

8. Lager nach Anspruch 6, bei dem das Adaptionsrohr (7) aus einem Material besteht, welches auf seiner Außenwandung versilbert ist.

9. Lager nach Anspruch 6, bei dem das Adaptionsrohr (7) aus einem Material besteht, welches auf seiner Außenwandung mit Hydroxylapatit beschichtet ist.

10. Lager nach Anspruch 6, bei dem das Adaptionsrohr (7) aus einem Material besteht, welches auf seiner Außenwandung mit Kalziumphosphat beschichtet ist.

11. Lager nach Anspruch 6, bei dem das Adaptionsrohr (7) aus einem Material besteht, welches auf seiner Außenwandung mit Titan beschichtet ist.

12. Lager nach Anspruch 6, bei dem das Adaptionsrohr (7) aus einem Material besteht, welches auf seiner Außenwandung mit Plasmatitanspray beschichtet ist.

13. Lager nach Anspruch 6, bei dem das Adaptionsrohr (7) aus Polyurethan besteht.

14. Lager nach einem der Ansprüche 6 bis 13, bei dem das Adaptionsrohr (7) eine solche Länge aufweist, dass es mit seiner distalen Stirnkante (9) auf einer Schulter (10) aufsitzt, die an der am Ankoppelungselement (6) angekoppelten extrakorporalen Koppelungseinrichtung (4) ausgebildet ist.

## Claims

1. Subcutaneous, intramuscular bearing for a rigid transcutaneous implant, which can be anchored intracorporeally in a bone stump, which has a spacer (3) between the implant and an extracorporeal coupling device (4) which can be coupled thereto, in which the spacer (3) is formed as a rigid bushing (5) having a coupling element (6) which is closed intracorporeally, to which the extracorporeal coupling device (4) can be coupled, **characterised in that** the bushing (5) widens significantly from its end (12) to be aligned extracorporeally to its end (13) to be aligned intracorporeally and has a smooth surface.

2. Bearing according to claim 1, in which the bushing has a base edge and the ratio of the length of the base edge of the bushing (5) at its end (12) to be aligned extracorporeally to the length at its end (13) to be aligned intracorporeally is between 1:1.2 to 1:2.

3. Bearing according to claim 1 or 2, in which the surface of the bushing (5) acts in anti-bacterial manner.

4. Bearing according to claim 3, in which the surface of the bushing (5) is silver-coated

5. Bearing according to claim 3, in which the surface of the bushing (5) is titanium-coated.

6. Bearing according to one of claims 1 to 5 having an adaptation tube (7) which engages into the interior of the bushing (5) and sits there releasably in a press fit, into which the coupling device (4), which can be coupled, can be placed, having anti-bacterial effect at least on its outer wall.

7. Bearing according to claim 6, in which the adaptation tube (7) consists of silver.

8. Bearing according to claim 6, in which the adaptation tube (7) consists of a material which is silver-coated on its outer wall.

9. Bearing according to claim 6, in which the adaptation tube (7) consists of a material which is coated with hydroxylapatite on its outer wall.

10. Bearing according to claim 6, in which the adaptation tube (7) consists of a material which is coated with calcium phosphate on its outer wall.

11. Bearing according to claim 6, in which the adaptation tube (7) consists of a material which is coated with titanium on its outer wall.

12. Bearing according to claim 6, in which the adaptation tube (7) consists of a material which is coated with plasma titanium spray on its outer wall.

13. Bearing according to claim 6, in which the adaptation tube (7) consists of polyurethane.

14. Bearing according to one of claims 6 to 13, in which the adaptation tube (7) has such a length that it sits with its distal end-face edge (9) on a shoulder (10), which is formed on the extracorporeal coupling device (4) coupled to the coupling element (6).

## Revendications

1. Support intramusculaire sous-cutané pour un implant transcutané rigide qui peut être ancré au niveau intracorporel dans un moignon osseux, qui présente une pièce intermédiaire (3) entre l'implant et un dispositif de couplage extracorporel (4) pouvant être couplé à celui-ci, la pièce intermédiaire (3) étant constituée comme une bague rigide (5) comportant un élément de couplage (6) fermé dans le sens intracorporel, auquel le dispositif de couplage extracorporel (4) peut être couplé,
**caractérisé en ce que**
la bague (5) s'élargit de façon importante de son extrémité (12) à aligner au niveau extracorporel à son extrémité (13) à aligner au niveau intracorporel et présente une surface lisse.

2. Support selon la revendication 1, dans lequel la bague présente une bordure de base et la longueur de la bordure de base de la bague (5) évolue à son extrémité à aligner au niveau extracorporel (12) par rapport à la longueur à son extrémité à aligner au niveau intracorporel (13) selon un rapport entre 1:1,2 à 1:2.

3. Support selon la revendication 1 ou 2, dans lequel la surface de la bague (5) a un effet antibactérien.

4. Support selon la revendication 3, dans lequel la surface de la bague (5) est argentée.

5. Support selon la revendication 3, dans lequel la surface de la bague (5) est recouverte de titane.

6. Support selon l'une quelconque des revendications 1 à 5, doté d'un tube adaptateur (7) qui est en prise avec l'intérieur de la bague (5) et y est inséré de façon amovible en un ajustage par pression dans lequel le dispositif de couplage (4) pouvant être couplé peut être placé, comportant un effet antibactérien au moins au niveau de sa paroi extérieure.

7. Support selon la revendication 6, dans lequel le tube adaptateur (7) est en argent.

8. Support selon la revendication 6, dans lequel le tube adaptateur (7) est constitué d'un matériau qui est argenté sur sa paroi externe.

9. Support selon la revendication 6, dans lequel le tube adaptateur (7) est constitué d'un matériau qui est revêtu d'hydroxylapatite sur sa paroi externe.

10. Support selon la revendication 6, dans lequel le tube adaptateur (7) est constitué d'un matériau qui est revêtu de phosphate de calcium sur sa paroi externe.

11. Support selon la revendication 6, dans lequel le tube adaptateur (7) est constitué d'un matériau qui est revêtu de titane sur la paroi externe.

12. Support selon la revendication 6, dans lequel le tube adaptateur (7) est constitué d'un matériau qui est revêtu d'un plasma de titane pulvérisé sur sa surface externe.

13. Support selon la revendication 6, dans lequel le tube adaptateur (7) est en polyuréthane.

14. Support selon l'une quelconque des revendications 6 à 13, dans lequel le tube adaptateur (7) présente une longueur telle qu'il est placé avec son bord de face (9) distal sur un épaulement (10) qui est formé au niveau du dispositif de couplage (4) extracorporel couplé à l'élément de couplage (6).
